# EUROPÄISCHE PATENTSCHRIFT

(11) **EP 2 355 807 B1**
(45) Veröffentlichungstag und Bekanntmachung des Hinweises auf die Patenterteilung: **22.08.2018**
(21) Anmeldenummer: 09804240.1
(22) Anmeldetag: 08.12.2009
(51) Int. Cl.: A61K 31/155, A61K 31/17, A61P 9/00, A61P 9/02, A61P 9/10, A61P 11/06, A61P 25/00, A61P 25/06, A61P 29/00, A61P 31/04, A61P 35/00, C07C 257/14, C07C 279/12

(54) **INHIBITOREN DER DIMETHYLARGININ DIMETHYLAMINOHYDROLASE**
INHIBITORS OF DIMETHYLARGININE DIMETHYLAMINOHYDROLASE
INHIBITEURS DE LA DIMÉTHYLARGININE DIMÉTHYLAMINOHYDROLASE

(30) Priorität: 10.12.2008 DE 102008061247
(43) Veröffentlichungstag der Anmeldung: 17.08.2011
(73) Patentinhaber: Christian-Albrechts-Universität zu Kiel, 24118 Kiel (DE)
(72) Erfinder: CLEMENT, Bernd, 24106 Kiel (DE); KOTTHAUS, Jürke, 24118 Kiel (DE); SCHADE, Dennis, 27632 Dorum (DE)
(74) Vertreter: Steinecke, Peter
(86) Internationale Anmeldenummer: PCT/DE2009/001724
(87) Internationale Veröffentlichungsnummer: WO 2010/066239

(56) Entgegenhaltungen:
- EP-A2- 0 330 629
- WO-A1-93/04048
- WO-A1-97/32844
- WO-A2-2006/051314
- ROBERTSON J G ET AL: "Inhibition of Bovine Brain Nitric Oxide Synthase by alpha-Amino and alpha-Carboxyl Derivatives of N-Allyl-L-arginine" BIOORGANIC CHEMISTRY, ACADEMIC PRESS INC., NEW YORK, NY, US LNKD- DOI:10.1006/BIOO.1995.1012, Bd. 23, 1. Januar 1995 (1995-01-01), Seiten 144-151, XP002374532 ISSN: 0045-2068
- KOTTHAUS J ET AL: "Structure-activity relationship of novel and known inhibitors of human dimethylarginine dimethylaminohydrolase-1: Alkenyl-amidines as new leads" BIOORGANIC & MEDICINAL CHEMISTRY, PERGAMON, GB LNKD- DOI:10.1016/J.BMC.2008.10.058, Bd. 16, Nr. 24, 15. Dezember 2008 (2008-12-15), Seiten 10205-10209, XP025685109 ISSN: 0968-0896 [gefunden am 2008-10-29]

## Beschreibung

Die vorliegende Erfindung betrifft neue Inhibitoren der Dimethylarginin Dimethylaminohydrolase (DDAH) nach Anspruch 1, zur Verwendung in der Behandlung der Ischämie-Reperfusion-Verletzung des Herzens oder Gehirns, Hypotonie, (chronischen) Entzündungen, neurodegenerativen Erkrankungen, Tumorerkrankungen, Peritonitis, Asthma, Migräne, Schmerz, Sepsis oder septischem Schock oder zur Prophylaxe von Schmerzen. Die DDAH katalysiert den physiologischen Abbau von *N*^{ω}-Monomethyl-L-arginin (NMMA) und *N*^{ω},*N*^{ω}-Dimethyl-L-arginin (ADMA) zu L-Citrullin und Mono- bzw. Dimethylamin. Diese *N*^{ω}-methylierten L-Arginine entstehen durch Methylierung von proteingebundenen Argininresten durch die Isoenzyme der Protein-Arginin-Methyltransferasen (PRMTs). Durch Proteinabbau werden diese beiden Substanzen freigesetzt und zirkulieren in physiologischen Konzentrationen von 0,4 bis 1,0 µM im Blut. In pathologischen Zuständen können die Werte jedoch deutlich ansteigen (bis zu 10 µM).

Sowohl ADMA als auch NMMA sind potente Inhibitoren aller drei Isoenzyme der Stickstoffmonoxid-Synthase (NOS), die die Freisetzung von Stickstoffmonoxid (NO) aus L-Arginin katalysieren. NO reguliert als potenter Vasodilatator den vaskulären Tonus, schützt vor atherosklerotischen Ereignissen und ist als Neurotransmitter an einer Vielzahl von Funktionen wie der Gedächtnisleistung oder der Ausbildung von Schmerzen beteiligt. Darüber hinaus nimmt NO eine wichtige Funktion in der unspezifischen Immunabwehr ein. Aufgrund dieser vielseitigen Funktionen von NO ist eine ausgewogene Regulation der NO-Biosynthese lebensnotwendig. Eine verringerte NO-Biosynthese kann zu kardiovaskulären Erkrankungen wie Hypertonie, Atherosklerose oder koronarer Herzkrankheit sowie zu erektiler Dysfunktion führen.

Darüber hinaus ist eine Vielzahl pathologischer Zustände mit einer Überproduktion von NO verbunden, so dass eine Hemmung der NO-Biosynthese zur Therapie dieser Krankheiten ausgenutzt werden kann. Beispiele für solche pathophysiologischen Zustände sind die Krankheitsbilder des septischen Schockes, des Schlaganfalls, neurodegenerativer Erkrankungen, chronischer Entzündungen, rheumatoider Arthritis, Migräne, Entzündungsschmerz (Nozizeption), Diabetes mellitus und Meningitis.

Obwohl in den vergangenen Jahren viele Versuche unternommen wurden, eine direkte Inhibition der NO-Synthase pharmazeutisch zur Therapie dieser Krankheitsbilder auszunutzen, hat noch keine der getesteten Verbindungen eine Zulassung als Arzneistoff erhalten.

Ein neuartiger Ansatzpunkt zur indirekten Inhibition der Isoenzyme der NO-Synthase stellt die Hemmung der DDAH dar. Die Inhibition der DDAH führt zu einem Anstieg der Blutspiegel von *N^{ω}*-methylierten L-Argininen und somit zu einer indirekten Hemmung der Aktivität der NOS. Mittlerweile konnte in mehreren Studien eine Beeinflussung der NO-Biosynthese durch eine Modulation der DDAH-Aktivität bestätigt werden. So wurde in Tiermodellen nachgewiesen, dass eine Hemmung der DDAH zu einem Anstieg der ADMA-Blutspiegel und des vaskulären Tonus führt (Rossiter et al. (2005) Selective substrate-based inhibitors of mammalian dimethylarginine dimethylaminohydrolase. Journal of Medicinal Chemistry 48, 4670-4678; Leiper et al. (2007) Nature Medicine 13: 198-203). Darüber hinaus kommen selektive DDAH-1-Inhibitoren zur Therapie von chronischen Schmerzen sowie des septischen Schockes in Frage (Leiper et al. (2007) Nature Medicine 13: 198-203). Als weitere Indikationsmöglichkeit kann eine Hemmung der DDAH in der Therapie von neurodegenerativen Erkrankungen wie zum Beispiel Morbus Alzheimer angenommen werde, da bei diesem Krankheitsbild sowohl verringerte ADMA-Blutspiegel als auch eine erhöhte DDAH-Aktivität nachgewiesen wurden.

In den vergangenen Jahren konnte eine Koexpression der DDAH-1 mit der nNOS sowie eine Kolokalisation der DDAH-2 mit der eNOS und zum Teil mit der iNOS nachgewiesen werden. Demzufolge kann eine selektive Hemmung der DDAH-1 möglicherweise zur selektiven Reduktion der Aktivität der nNOS ausgenutzt werden. Inzwischen wurde der Einsatz von selektiven DDAH-1 Inhibitoren erfolgreich *in vivo* zur Therapie von chronischen Schmerzen und des septischen Schocks getestet. Des Weiteren wurde eine Beteiligung der DDAH-1 am Tumorwachstum und der Angiogenese nachgewiesen. Tumorgewebe mit erhöhter DDAH-1 Expression proliferierte wesentlich schneller als andere Tumorgewebe.

Die Hemmung der DDAH-2 hingegen ist wenig sinnvoll, da eine verminderte endotheliale NO-Freisetzung einen Risikofaktor für kardiovaskuläre Komplikationen darstellt.

Bisher bekannte Inhibitoren für die humane DDAH-1 stellen die L-Arginin-Derivate dar. Der derzeit potenteste Vertreter dieser Gruppe ist das *N*^{ω}-(2-Methoxyethyl)-L-arginin (L-257, IC₅₀ 22 µM, murine DDAH) ein Guanidin (Rossiter et al. (2005) Selective substrate-based inhibitors of mammalian dimethylarginine dimethylaminohydrolase. Journal of Medicinal Chemistry 48: 4670-4678). Darüber hinaus stellen die *N^{δ}*-(1-Iminoalk(en)yl-L-ornithine eine sehr potente Inhibitorenklasse mit Vinyl-L-NIO als derzeit potentesten bekannten Inhibitor der DDAH-1 dar (Kotthaus et al. 2008 Bioorganic & Medicinal Chemistry 16: 10205-10209).

Es ist bekannt die orale Bioverfügbarkeit der Guanidine und Amidine mittels Prodrug-Prinzipien zu erhöhen. Ein sehr erfolgreiches Prinzip stellen dabei die *N*-Hydroxyamidine (Amidoxime) und *N*-Hydroxyguanidine [Clement, B. DE4321444 und PCT/DE2007/001216] dar.

Die bisher bekannten Inhibitoren für die humane DDAH-1 besitzen aufgrund der Aminosäure-Struktur schlechte pharmakokinetische Eigenschaften, da sie bei physiologischem pH-Wert als Zwitterion vorliegen (Rossiter et al. (2005) Selective substrate-based inhibitors of mammalian dimethylarginine dimethylaminohydrolase. Journal of Medicinal Chemistry 48: 4670-4678). Die Aminofunktion ist protoniert und somit positiv geladen während die Carboxylfunktion deprotoniert und somit negativ geladen ist. Geladene Substanzen werden nur in einem geringen Umfang aus dem Gastrointestinaltrakt resorbiert, da sie die lipophilen Membranen des Gastrointestinaltraktes nicht durch Diffusion durchdringen können (Ettmayer et al. (2004) Lessons learned from marketed and investigational prodrugs J. Med. Chem. 47: 2393-2404, Anand et al. (2002) Current prodrug strategies via membrane transporters/receptors Expert Opin. Biol. Ther. 2: 607-620). Die passive Diffusion stellt jedoch den bevorzugten Absorptionsmechanismus eines Arzneistoffes nach oraler Gabe dar. Somit ist das Erreichen wirksamer Substanz-Spiegel im Blut nach oraler Gabe geladener Verbindungen sehr unwahrscheinlich. Darüber hinaus besteht auch die Möglichkeit der Aufnahme über spezifische Transportsystem wie die Aminosäure-Transporter, jedoch ist der Umfang der Resorption durch diesen Mechanismus deutlich geringer einzuschätzen (Anand et al. (2002) Current prodrug strategies via membrane transporters/receptors. Expert Opin. Biol. Ther. 2: 607-620). Aus diesem Grund ist das Entfernen von Ladungen aus einem Molekül von eminenter Bedeutung für die Entwicklung von Arzneistoffen, die eine orale Bioverfügbarkeit aufweisen sollen (Clement et al. (2006) Diacetyldiaminoximester of pentamidine, a prodrug for treatment of protozoal diseases: synthesis, in vitro and in vivo biotransformation. Chem. Med. Chem. 1: 1260-1267).

WO 2006/051314 A2 beschreibt Verbindungen die als DDAH-Inhibitoren nützlich sind. WO 93/04048 bezieht sich auf die Pyrazin-Derivate und EP 0330629 A2 auf Guanidin-Derivate mit hypotensiver Aktivität. Robertson et al. (1995) Bioorganic Chemistry 23: 144-151 beschreibt Inhibierung von Rinderhirnstickstoffoxidsyntase mit α-Amino und α-Carboxyl-Derivativen vom *N^{G}*-Allyl-L-Arginin. WO97/32844 bezieht sich auf Hydroxyamidin-Derivate, die als Stickstoffoxidsynthaseinhibitoren nützlich sind. Kotthaus et al. (2008) Bioorganic & Medicinal Chemistry 16: 10205-10209 bezieht sich auf Struktur-Aktivität-Verbindung von humanen Dimethylarginindimethylamininhydrolase-1 Inhibitoren.

Daher ist es Aufgabe der Erfindung neue spezifische Inhibitoren der humanen DDAH-1 zur Verfügung zu stellen, die verbesserte pharmakokinetische Eigenschaften haben.

Die Aufgabe wird gelöst durch die Merkmale des Anspruchs 1. Die Unteransprüche geben vorteilhafte Ausgestaltungen der Erfindung an.

### Beschreibung der Erfindung

Im Rahmen der Erfindung wurden neue Inhibitoren der humanen DDAH-1 synthetisiert und auf ihre Wirksamkeit getestet. Die erfindungsgemäßen Inhibitoren werden durch folgende allgemeine Strukturformel dargestellt: 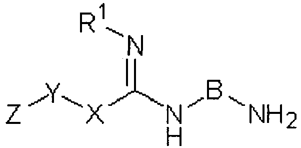 wobei
- B: eine verzweigte oder unverzweigte, substituierte oder nicht substituierte, gesättigte oder ungesättigte Kohlenwasserstoffkette mit einer Kettenlänge von 1 bis 6 und/oder ein substituiertes oder nicht substituierte aromatisches System mit einer Ringgröße von 3 bis 6 ist,
- R1: den Strukturen (i-vii) entspricht: 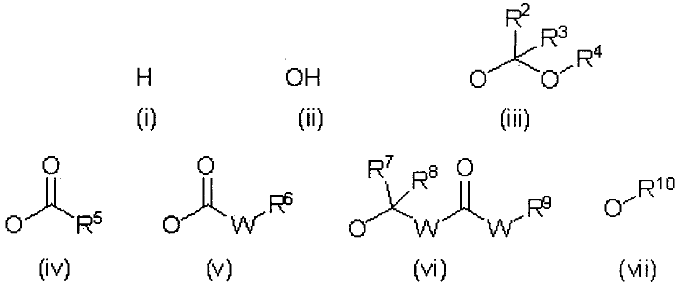 wobei
R2-4 einem Wasserstoff, Alkyl- oder Arylresten entsprechen können oder so gestaltet sind, dass sie in ihrer Struktur einem Monosaccharid(derivat) entsprechen, R5-6 einem Wasserstoff, einer Kohlenwasserstoffkette mit einer Kettenlänge von 1 bis 8 oder einem Arylrest entsprechen können, R7-10 einem Wasserstoff, Alkyl- oder Arylresten entsprechen können und W einem Sauerstoff- oder einem Stickstoff-Atom entspricht,
- X: Kohlenstoff (C) oder Stickstoff (N) ist,
- Y: eine verzweigte oder unverzweigte, substituierte oder nicht substituierte, gesättigte oder ungesättigte Kohlenwasserstoffkette mit einer Kettenlänge von 1 bis 6 und/oder ein substituiertes oder nicht substituierte aromatisches System mit einer Ringgröße von 3 bis 6 ist,
- Z: Wasserstoff oder eine Methoxylgruppe ist,
und
der die Aminogruppe tragende Rest (B) keine Carboxylgruppe aufweist,
wobei mögliche Substituenten für Y ausgewählt sein können aus Halogen (Fluor, Chlor, Brom und Jod), Sauerstoff, Schwefel, Alkoxy, Acyloxy, Amino, Hydroxyl, Carbonyl, Mercapto, Carboxy, Benzyloxy, Phenyl, Benzyl, Cyano, Nitro, Thioalkoxy, Carboxyaldehyd, Carbalkoxy und Carboxamid, und wobei ein Substituent von B ausgewählt ist aus der Gruppe bestehend aus Halogen (Fluor, Chlor, Brom und Jod), Schwefel, Alkoxy, Amino, Hydroxyl, Carbonyl, Mercapto, Benzyloxy, Phenyl, Benzyl, Cyano, Nitro und Thioalkoxy-Gruppe, sowie die Salze der Verbindungen.

Es versteht sich, dass, wenn X Kohlenstoff (C) oder Stickstoff (N) ist, anstelle von X eine Methylengruppe (-CH2-) oder eine sekundäre Aminogruppe (-NH-) vorhanden ist.

Ein Vergleich mit der bereits als Inhibitor der humanen DDAH bekannten Verbindung L-257 zeigt dabei, dass erstmalig auch Verbindungen ohne α-Carboxylfunktion, wie sie in Aminosäuren vorliegt, ebenfalls eine potente Hemmung der hDDAH-1 bewirken. Die Allgemeinheit der Lehre nicht einschränkend zeigt Tabelle 1 eine Übersicht der Hemmwerte auf die hDDAH-1. Die dargestellten Daten für die Hemmung bei 1 mM sind MW ± SD von mindestens vier separaten Inkubationen, die jeweils doppelt vermessen wurden. Die IC50- und Ki-Wert Bestimmung wurde mindestens doppelt durchgeführt. n.b., nicht bestimmt.

**Tabelle 1**

| | | | | |
|---|---|---|---|---|
| | | 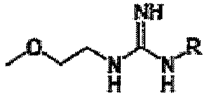 | | |

| Substanz | R | Hemmung bei 1 mM [%] | IC₅₀ [µM] | *K*ᵢ [µM] |
|---|---|---|---|---|
| L-257 | 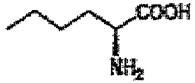 | 89 ± 2 | 29 ± 7 | 13 ± 2 |
| CbMEG | 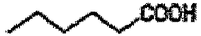 | 42 ± 6 | n.b. | n.b. |
| 3 | 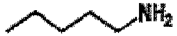 | 80 ± 5 | 70 ± 16 | 18 ± 6 |

Die Erkenntnis, dass die α-Carboxylfunktion nicht essentiell ist, ist beachtlich, denn die Kristallstruktur der DDAH-1 unterstreicht die Bedeutung der α-Carboxylfunktion von Aminosäuren, da von ihr drei ionische Wechselwirkungen zum aktiven Zentrum der DDAH-1 ausgebildet werden (Murray-Rust et al. (2001) Structural insights into the hydrolysis of cellular nitric oxide synthase inhibitors by dimethylarginin dimethylaminohydrolase. Nature Structural Biology 6: 679-683). Das erfindungsgemäße Ausführungsbeispiel 3 zeigt eine potente Hemmung der DDAH-1 und besitzt aufgrund des Verlustes der α-Carboxylfunktion im Vergleich zu L-257 durch den Verlust der Carboxylfunktion verbesserte pharmakokinetische Eigenschaften auf, so dass die Wahrscheinlichkeit einer Absorption aus dem Gastrointestinaltrakt erhöht ist.

Die Erkenntnis, dass die α-Carboxylfunktion nicht essentiell ist, wurde auf *N^{δ}*-(1-Iminoalk(en)yl)-L-ornithine übertragen (Tabelle 2). Auch Ausführungsbeispiel 5 zeigt eine Hemmung der DDAH-1, so dass eine Entwicklung von Inhibitoren ohne α-Carboxylfunktion ebenfalls auf Amidin-Basis möglich ist.

**Tabelle 2**

| Substanz | | Hemmung bei 1 mM [%] |
|---|---|---|
| 5 | 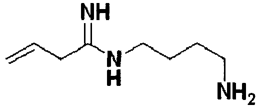 | 40 ± 6 |

Die dargestellten Daten für die Hemmung bei 1 mM sind MW ± SD von mindestens vier separaten Inkubationen, die jeweils doppelt vermessen wurden.

Diese Erkenntnisse zeigen überraschenderweise erstmals, dass die Entwicklung potenter Inhibitoren ohne α-Carboxylfunktion, die somit nicht mit den pharmakokinetischen Nachteilen der Aminosäuren versehen sind, möglich ist.

Zur Verbesserung der oralen Bioverfügbarkeit der erfindungsgemäßen Inhibitoren der humanen DDAH-1 können dabei bekannte Prodrug-Prinzipien für Amidine und Guanidine insbesondere *N*-Hydroxyamidine (Amidoxime) und *N*-Hydroxyguanidine entsprechend [Clement, B. Methoden zur Behandlung und Prophylaxe der Pneomocystis carinii Pneumonie (PCP) und anderen Erkrankungen sowie Verbindungen und Formulierungen zum Gebrauch bei besagten Methoden. P. 432444.4, 1993] zum Einsatz kommen.

Die erfindungsgemäßen Inhibitoren der humanen DDAH-1 können generell zur Therapie von Krankheiten und pathophysiologischen Zuständen eingesetzt werden, die auf eine Überproduktion von NO zurückzuführen sind. Als Indikationen kommen der septische Schock, chronische oder nicht chronische Schmerzen, Schlaganfall, chronische oder nicht chronische Entzündungen, rheumatoide Arthritis, Migräne, Asthma, Ischämie-Reperfusion-Verletzung des Herzens oder Gehirns, Hypotonie und neurodegenerative Erkrankungen in Frage. Insbesondere bei Alzheimer Patienten wurden geringere ADMA-Blutspiegel nachgewiesen als in gesunden Kontrollgruppen, so dass hier eine Therapie mit DDAH-Inhibitoren erfolgsversprechend erscheint. Darüber hinaus können DDAH-Inhibitoren zur Therapie von Tumorerkrankungen, Sepsis oder Peritonitis angewendet werden.

### Material und Methoden

### Ausführungsbeispiel A: N-(4-Aminobutyl)-N'-(2-methoxyethyl)guanidin N-Benzyloxycarbonyl-N'-(2-methoxyethyl)-thioharnstoff (1)

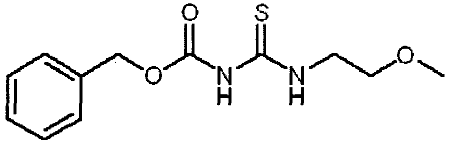

Allgemeine Vorschrift in Anlehnung an Linton, Carr et al. 2000:
7,0 mmol 2-Methoxyethylamin werden in 250 mL trockenem Dichlormethan gelöst. Die Lösung wird auf 0 °C gekühlt und 14 mL einer 0,5 M Lösung (in Dichlormethan) Benzyloxycarbonylisothiocyanat (7,0 mmol) tropfenweise über einen Zeitraum von 30 min zugegeben. Das Reaktionsgemisch wird zwei Stunden gerührt, wobei die Lösung auf Raumtemperatur aufwärmt. Anschließend wird das Gemisch am Rotationsverdampfer auf ca. ein Drittel des ursprünglichen Volumens i. Vak. eingeengt und mit jeweils 25 mL 1 %-iger HCl, Wasser und gesättigter NaCl-Lösung gewaschen. Die organische Phase wird über Na₂SO₄ getrocknet und am Rotationsverdampfer entfernt. Der Thioharnstoff **1** sind an dieser Stelle meist schon zu > 96 % rein (DC) und wird durch Kristallisation weiter aufgereinigt. Das nach dem Einrotieren erhaltene Rohprodukt kristallisiert unter starker Wärmeentwicklung spontan aus. Es wird mit wenig kaltem Cyclohexan angerieben, filtriert und mit kleinen Portionen kaltem Cyclohexan gewaschen und getrocknet.

| | |
|---|---|
| Ausbeute: | 1,76 g weiße Kristalle (94 %) |
| DC: | R_{f} = 0,30 (Cyclohexan/Ethylacetat, 4:1) |
| Schmp.: | 88,5 °C |

¹H-NMR (CDCl₃):
   δ/ppm = 3.39 (s, 3H, O-CH₃), 3.60 (t, ³*J* = 5.2 Hz, 2H, O-CH₂), 3.86 (q, ³*J* = 5.2 Hz, 2H, N-CH₂), 5.19 (s, 2H, CH₂-Cbz), 7.32-7.61 (m, 5H, ArH), 8.11 (br s, 1H, NH), 9.84 (br s, 1H, NH).
¹³C-NMR (CDCl₃):
   δ/ppm = 46.2 (N-CH₂), 59.6 (O-CH₃), 68.8 (O-CH₂), 70.4 (O-CH₂), 129.0, 129.4, 129.5 (ArCH), 135.2 (ArC), 153.0 (CO), 179.9 (C=S).
MS (ESI):
   m/z = 269 [M + H]⁺, 225 [M - CO₂ + H]⁺, 91 [C₇H₇]⁺.
   C₁₂H₁₆N₃O₂S (268.33)
   Ber. C 53.71 H 6.01 N 10.44
   Gef. C 53.93 H 6.19 N 10.61

### N-Benzyloxycarbonyl-N'-[4-(t-butyloxycarbonyl)aminobutyl]-N"-(2-methoxyethyl)guanidin (2)

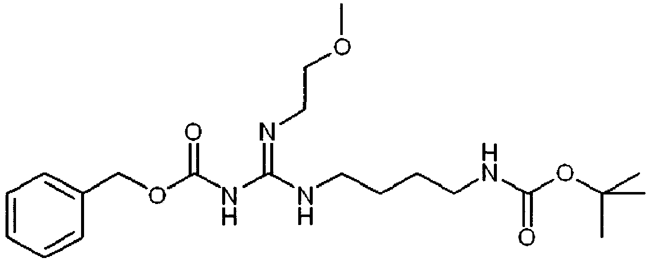

Allgemeine Vorschrift in Anlehnung an Linton, Carr et al. 2000:
Es werden 0,5 mmol des Thioharnstoffs **1** in 5 mL trockenem Dichlormethan gelöst und 261 µL DIPEA (1,5 mmol) sowie 1,5 mmol *N*-Boc-1,4-diaminobutan zugegeben. Die Lösung wird für ca. 30 min auf 0 °C gebracht und 287 mg EDCI (1,5 mmol) zugegeben. Sofern nicht anders angemerkt, wird das Gemisch über Nacht bei Raumtemperatur gerührt. Die Lösung wird mit ca. 10 mL Dichlormethan verdünnt und jeweils mit 5 mL 1 %-iger HCl, Wasser und gesättigter NaCl-Lösung gewaschen. Die organische Phase wird über Na₂SO₄ getrocknet und am Rotationsverdampfer eingeengt. Es resultieren meist Öle, die mittels Flashchromatographie über Kieselgel weiter aufgereinigt werden. Die verwendeten Elutionsmittel und erzielten Ausbeuten sind bei den jeweiligen Substanzen angegeben. Als Elutionsmittel wird Dichlormethan/Methanol (95:5) verwendet.

| | |
|---|---|
| Ausbeute: | 198 mg eines farblosen Öls (94 %) |
| DC: | R_{f} = 0,41 (Dichlormethan/Methanol, 96:4; Reagenz A und C) |

¹H-NMR (CDCl₃):
   δ/ppm = 1.36 (s, 9H, C(CH₃)₃), 1.41-1.57 (m, 4H, N-CH₂-CH₂-CH₂), 3.06, 3.19 (2 × m, 2H, N-CH₂), 3.30 (s, 3H, O-CH₃), 3.31-3.44 (m, 4H, N-CH₂-CH₂-O), 4.56 (br s, 1H, NH), 5.04 (s, 2H, CH₂-Cbz), 7.24-7.36 (m, 3H, ArH), 7.39-7.43 (m, 2H, ArH), 9.19 (br s, 1H, NH).
¹³C-NMR (CDCl₃):
   δ/ppm = 27.1, 28.2 (N-CH₂-CH₂-CH₂), 29.1 (C(CH₃)₃), 40.7, 41.4 (3 × N-CH₂), 59.6 (O-CH₃), 67.2 (O-CH₂, CH₂-Cbz), 80.0 (C(CH₃)₃), 128.2, 128.6, 128.9 (ArCH), 138.5 (ArC), 156.7 (C=N), 164.8 (CO).
MS (ESI):
   m/z = 423 [M + H]⁺.

### N-(4-Aminobutyl)-N'-(2-methoxyethyl)guanidin Bis(trifluoracetat) (3)

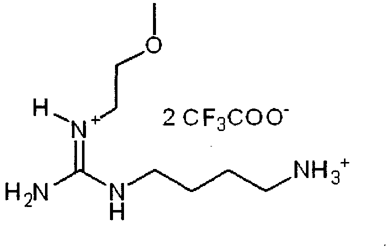

Das geschützte Guanidin **2** (0,5 mmol) wird mit 10 mL TFA und 3 mL Thioanisol über Nacht bei Raumtemperatur gerührt. Anschließend wird der Großteil an TFA i. Vak. abdestilliert und 5 mL Wasser sowie 15 mL Diethylether werden zugegeben. Die organische Phase wird noch zweimal mit 5 mL Wasser extrahiert und die vereinigten wässrigen Phasen abschließend mit 5 mL Diethylether gewaschen. Die wässrige Phase wird i. Vak. einkonzentriert und chromatographisch aufgereinigt. Das Rohprodukt wird mittels Flashchromatographie über eine RP-18-Säule gereinigt (Elutionsmittel: 0,1 % TFA_{(aq)}). Die Ninhydrin-positiven Fraktionen werden vereinigt und i. Vak. einkonzentriert.

| | |
|---|---|
| Ausbeute: | 234 mg eines klaren Öls (99 %) |
| DC: | R_{f} = 0,22 (*i*-Propanol/Wasser/Eisessig, 8:2+0,5; Reagenz A) |

¹H-NMR (DMSO-*d*₆):
   δ/ppm = 1.54 (m, 4H, N-CH₂-CH₂-CH₂), 2.80 (m, 2H, N-CH₂), 3.14 (m, 2H, N-CH₂), 3.27 (s, 3H, O-CH₃), 3.32, 3.42 (2 × t, 4H, N-CH₂-CH₂-O), 7.47 (br s, 2H, NH₂), 7.61 (br t, 1H, NH), 7.71 (br t, 1H, NH), 7.87 (br s, 3H, NH₃⁺).
¹³C-NMR (DMSO-*d*₆):
   δ/ppm = 24.1 (CH₂-CH₂-NH₃⁺), 25.4 (CH₂-CH₂-CH₂-NH₃⁺), 38.3 (N-CH₂), 40.3 (N-CH₂), 40.7 (N-CH₂), 58.0 (O-CH₃), 70.0 (O-CH₂), 155.9 (C=N).
MS (ESI):
   m/z = 189 [M + H]⁺.
   C₈H₁₆N₄O₅·2,5 CF₃COOH (473.33)
   Ber. C 32.99 H 4.79 N 11.84
   Gef. C 32.86 H 4.80 N 11.50

### Ausführungsbeispiel B: N-(4'-Aminobutyl)but-3-enamidin N-[4'-(t-Butyloxycarbonyl)-aminobutyl]but-3-enamidin Hydrochlorid (4)

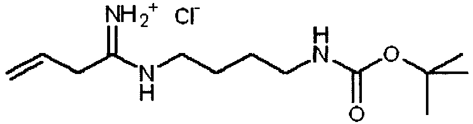

Es werden 188 mg *N*-(*t*-Butyloxycarbonyl)-1,4-diaminobutan (1 mmol) und 407 mg But-3-enimidsäuremethylester Hydrochlorid (3 mmol) in 10 mL Wasser bei 0 °C gelöst. Zu dieser Lösung wird 2,5 M NaOH zugegeben bis ca. pH 10 erreicht ist und anschließend zwei Stunden bei 0 °C, sowie eine Stunde bei Raumtemperatur gerührt. Das Reaktionsgemisch wird mit verdünnter HCl auf pH 7 gebracht und über Nacht bei Raumtemperatur gerührt. Der Ansatz wird bei ca. 30 °C am Rotationsverdampfer einkonzentriert und mittels Säulenchromatographie über Kieselgel aufgearbeitet, wobei ein Ethylacetat/Methanol-Gradient (stufenweise: 20-40 % Methanol) verwendet wird.

| | |
|---|---|
| Ausbeute: | 250 mg eines weißgetrübten Öls (86 %) |
| DC: | R_{f} = 0,17 (Ethylacetat/Methanol, 8:2; Reagenz A) |

¹H-NMR (DMSO-*d*₆):
   δ/ppm = 1.36 (s, 9H, C(CH₃)₃), 1.39-1.54 (m, 4H, 2',3'-CH₂), 2.91 (m, 2H, 4'-CH₂), 3.18-3.29 (m, 4H, 2,1'-CH₂), 5.20 (dd, ³*J*_{cis} = 10.0 Hz, ²*J* = 1.3 Hz, 1H, CH=CH₂), 5.30 (dd, ³*J*ₜᵣₐₙₛ = 17.0 Hz, ²*J* = 1.5 Hz, 1H, CH=CH₂), 5.91 (ddt, ³*J* = 6.8, 10.0, 17.0 Hz, 1H, CH=CH₂), 6.81, 8.87, 9.40, 9.98 (4 × br s, 1H, NH).
¹³C-NMR (DMSO-*d*₆):
   δ/ppm = 24.6, 26.6 (2',3'-CH₂), 28.2 (C(CH₃)₃), 36.0 (2-CH₂), 39.2, 41.5 (1',4'-CH₂), 77.3 (C(CH₃)₃), 119.7 (CH=CH₂), 130.9 (CH=CH₂), 155.5 (CO), 164.7 (C=N).
MS (ESI):
   m/z = 278 [M + Na]⁺, 256 [M + H]⁺, 200 [M - C₂H₈ + H]⁺.

### N-(4'-Aminobutyl)but-3-enamidin Bis(trifluoracetat) (5)

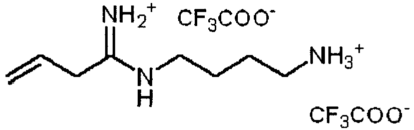

220 mg des Boc-geschützten Amidins 4 (0,75 mmol) werden in 5 mL TFA/Dichlormethan (1:1) für 30 min bei Raumtemperatur gerührt. Der Ansatz wird i. Vak. einkonzentriert, mit 5 mL Wasser versetzt und zweimal mit Diethylether gewaschen. Die Lösung wird bis auf ca. 1 - 2 mL einrotiert und mittels Flashchromatographie über eine RP-18-Säule weiter aufgearbeitet. Als Elutionsmittel wird 0,1 %-ige TFA in *Aqua bidest.* verwendet.

| | |
|---|---|
| Ausbeute: | 285 mg eines farblosen Öls (99 %) |
| DC: | R_{f} = 0,35 (*i*-Propanol/Wasser/Eisessig, 8:2+0,5; Reagenz A) |

¹H-NMR (DMSO-*d*₆):
   δ/ppm = 1.57 (m, 4H, 2',3'-CH₂), 2.81 (m, 2H, 4'-CH₂), 3.17-3.26 (m, 4H, 2,1'-CH₂), 5.23-5.31 (m, 2H, CH=CH₂), 5.87 (ddt, ³*J* = 17.0, 10.0, 6.7 Hz, 1H, CH=CH₂), 7.86 (br s, 3H, NH₃⁺), 8.74, 9.16, 9.57 (3 × br s, 1H, NH).
¹³C-NMR (DMSO-*d*₆):
   δ/ppm = 24.1, 24.2 (2',3'-CH₂), 36.5 (2-CH₂), 38.2, 41.2 (1',4'-CH₂), 120.0 (CH=CH₂), 130.4 (CH=CH₂), 165.1 (C=N).
MS (ESI):
   m/z = 156 [M + H]⁺, 139 [M - NH₃ + H]⁺.
   C₈H₁₇N₃·2,5 CF₃COOH·0,5 H₂O (449.31)
   Ber. C 34.75 H 4.60 N 9.35
   Gef. C 34.92 H 4.75 N 9.10

### Konstruktion des Expressionsplasmids für hDDAH-1

cDNA von humaner DDAH-1 wurde vom RZPD (Berlin, Deutschland) erworben und per PCR amplifiziert. Sense-Primer: 5'-AAGGATCCATGGCCGGGCTCGGCCAC-3' (mit einer Schnittstelle für BamHI (unterstrichen); Anti-Sense: 5'-GGAAGCTTGCAGCTCAGGAGTCT-3' (mit einer Schnittstelle für HindIII (unterstrichen). Für die PCR-Amplifikation wurde folgendes Cycler-Programm verwendet:

| | | T [°C] | Zeit [s] | | |
|---|---|---|---|---|---|
| 1. | Denaturierung | 94 | 180 | 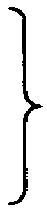 | |
| 2. | Denaturierung | 94 | 30 | | 28 Zyklen |
| | Annealing | 45 | 45 | | |
| | Extension | 72 | 180 | | |
| 3. | Extension | 72 | 900 | | |
| 3. | Hold | 4 | ∞ | | |

Das PCR-Produkt wurde in den Expressionsvektor pQE-30 (Qiagen, Hilden, Deutschland) unter Verwendung der Schnittstellen BamHI und HindIII ligiert.

Expression und Aufreinigung von His-tagged humaner DDAH-1 Für die Enzym-Expression wurde das Expressionsplasmid in BL-21 Zellen transformiert und eine Übernachtkultur in Kanamycin- und Ampicillin-haltigem LB-Medium bei 37 °C angelegt. Am Tag der Expression wurden ein Liter LB-Medium mit 50 ml der Übernachtkultur angeimpft und bei 30 °C inkubiert bis eine OD₆₀₀ von 0,6 erreicht wurde. Die Expression wurde mit Isopropyl-ß-thiogalactopyranosid (IPTG) in einer Konzentration von 100 µM induziert. Die Expressionszeit betrug zwischen 4 und 6 h bei einer Temperatur von 30 °C. Nach Abbruch der Expression wurden die Zellen abzentrifugiert (4.500 g, 4 °C, 10 min). Das Pellet wurde zur sofortigen Verwendung in 26 ml Zell-Lyse-Puffer (20 mM K₂HPO₄, 150 mM NaCl, 2 mM 2-Mercaptoethanol, 1 mM Phenylmethylsulfonylfluorid und 1 mM Benzamidin) suspendiert und die Zellen mit Hilfe einer French Press bei 19.000 psi (4 °C) lysiert. Dieser Vorgang wurde zweimal wiederholt. Im Anschluss wurden die groben Zellfragmente bei einer Zentrifugation (4.500 g, 4 °C, 10 min) abgetrennt und der Überstand mittels Ultrazentrifugation bei 33.000 g für 45 min bei 4 °C von den feineren Zelltrümmern befreit.

Der Überstand wurde mit 300 µl Ni²⁺-NTA-Matrix versetzt. Das Protein wurde bei 4 °C über 1,5 h bei leichtem Schütteln an die Matrix gebunden. Anschließend wurde das Protein mit Imidazol-haltigen Lösungen (50 mM Kaliumphosphat Puffer pH 7.0, 300 mM NaCl, 0.5 mM 2-Mercaptoethanol, 1 mM EDTA, 1 mM Benzamidin, 1 mM Phenylmethylsulfonylfluorid und 10% Glycerol) gewaschen bzw. das Protein nach folgendem Schema eluiert:
- Waschen mit 2 ml Waschlösung
- Waschen mit 2 ml Waschlösung (+10 mM Imidazol)
- Waschen mit 1 ml Waschlösung (+20 mM Imidazol)
- Elution mit 1 ml Waschlösung (+100 mM Imidazol)

### In vitro hDDAH-1-Assay (HPLC)

Dieser Assay wurde verwendet zur Bestimmung der Hemmwirkung der Inhibitoren in einer Konzentration von 1 mM. Ein Standard-Inkubationsansatz bestand aus 75 µl 50 mM Kaliumphosphat-Puffer pH 7,4 und enthielt NMMA in einer Konzentration von 200 µM. Die Inhibitoren wurden in Endkonzentrationen von 1 mM eingesetzt. Durch Zugabe von 2 µg hDDAH-1 wurde die Reaktion gestartet und die Proben für 30 min bei 37 °C im Schüttelwasserbad inkubiert. Im Anschluss wurde die Reaktion durch Zugabe von 75 µl Acetonitril abgestoppt, die Proben für 5 min geschüttelt und zentrifugiert (12.000 g, 5 min). Der Überstand wurde der HPLC zugeführt.

Zusätzlich wurden jeweils drei Inkubationen ohne Inhibitorzusatz durchgeführt und wie oben angegeben aufgearbeitet. Die in diesen Proben detektierte L-Citrullin-Konzentration wurde gleich 100 % gesetzt und die gebildeten Mengen L-Citrullin in den Proben mit Inhibitorzusatz auf diesen Wert bezogen.

### IC₅₀-Wert Bestimmung (Plattenreader-Assay)

Die Proben für den Plattenreader-Assay wurden direkt auf einer 96-well Mikrotiterplatte inkubiert. Ein Inkubationsansatz bestand aus 150 µl 50 mM Kaliumphosphat-Puffer pH 7,4 mit NMMA in einer Endkonzentration von 300 µM. Der Inhibitor wurde in fünf verschiedenen Konzentrationen zugegeben und die Reaktion durch Zugabe von 4 µg rekombinanter hDDAH-1 gestartet. Zusätzlich wurden zwei Ansätze ohne Inhibitorzusatz inkubiert. Die Platte wurde mit einem Deckel verschlossen und über 30 min auf einem Platten-Inkubator (Schüttelinkubator DTS-4, LTF-Labortechnik GmbH, Wasserburg, Deutschland) bei 37 °C inkubiert. Die Reaktionen wurden mit 200 µl des Colder-Reagenzes abgestoppt und das gebildete Citrullin derivatisiert (siehe Derivatisierung für Plattenreader-Assay). Anschließend wurde der IC₅₀-Wert mittels SigmaPlot 8.0 (SPSS Inc., Chicago, USA) berechnet.

### Kᵢ-Wert Bestimmung (Plattenreader-Assay)

Die *K*ᵢ-Wert Bestimmung erfolgte, wie für die IC₅₀-Wert Bestimmung beschrieben, mit dem Unterschied, dass fünf verschiedene NMMA-Konzentrationen (50, 100, 300, 750 und 1250 µM) eingesetzt wurden. Die Inkubationen wurden so durchgeführt, dass jede NMMA-Konzentration jeweils doppelt mit jeder der fünf Inhibitor-Konzentrationen inkubiert wurde. Darüber hinaus wurden Inkubationen mit den verschiedenen NMMA-Konzentrationen ohne Inhibitorzusatz durchgeführt.

Für jede Inhibitor-Konzentration wurde mit SigmaPlot 8.0 (SPSS Inc., Chicago, USA) der jeweilige apparente *K*ₘ-Wert sowie *V*ₘₐₓ bestimmt. Über eine Sekundärauftragung der Inhibitor-Konzentration gegen *K*ₘ/*V*ₘₐₓ wurde durch Extrapolation der Ausgleichsgeraden auf die Abszisse der *K*ᵢ-Wert ermittelt.

### Derivatisierung für Plattenreader-Assay

Für die Derivatisierung von Citrullin wurden zwei Reagenz-Lösungen hergestellt:

| **Lösung A** | | **Lösung B** | |
|---|---|---|---|
| 80 mM | Diacetylmonoxim | 200 ml | 85 % H₃PO₄ |
| 2 mM | Thiosemicarbazid | 330 ml | 96 % H₂SO₄ |
| | | 750 mg | NH₄Fe(SO₄)₂ x 12 H₂O |
| | | ad 1000 ml | *Aqua bidest.* |

Das Derivatisierungs-Reagenz (Colder-Reagenz) wurde unmittelbar vor der Derivatisierung durch Mischen von einem Teil Lösung A mit drei Teilen Lösung B hergestellt und vor direkter Sonneneinstrahlung geschützt aufbewahrt.

Die Derivatisierung erfolgte auf besonders hitzestabilen 96-well Mikrotiterplatten. Die Proben (150 µl) wurden mit 200 µl des Colder-Reagenz versetzt und die Platte zur Verhinderung von Verdunstung mit einer hitzestabilen Klebefolie (Nunc, Wiesbaden, Deutschland) verschlossen. Im Anschluss erfolgte die Farbentwicklung im Trockenschrank bei 95 °C über 15 - 20 min. Vor dem photometrischen Vermessen wurde gewartet bis die Platte Raumtemperatur erreicht hatte und dann die Farbintensität bei 540,5 nm (Carry 50 Microplatereader, Varian, Darmstadt, Deutschland) gemessen. Das Vermessen erfolgte innerhalb von 20 min, da das Reaktionsprodukt nicht stabil ist (Knipp und Vasak 2000).

### HPLC-Methode

Für die Analyse der Proben des *In vitro* hDDAH-1-Assays (HPLC) wurde eine o-PA-Vorsäulenderivatisierung mit anschließender Fluoreszenzdetektion der Aminosäuren eingesetzt.

| | | | |
|---|---|---|---|
| HPLC-Anlage: | Waters Autosampler 717plus, Waters 600 Controller, Waters 600 Pump, Waters Fluorescence Detector 470 und EZChrom Elite Client/Server Aufnahme- und Auswertesoftware (Version 2.8.3) | | |
| Stationäre Phase: | NovaPak RP-18-Säule 4 µm (4 x 150 mm, VDS Optilab) Vorsäule: Phenomenex C18 (4 x 3,0 mm) | | |
| Temperatur: | 30 °C | | |
| Mobile Phase: | Eluent A: | 10 mM Kaliumphosphat-Puffer pH 4,65 | 86 % |
| | | Acetonitril | 8 % |
| | | Methanol | 6 % |
| | Eluent B: | Acetonitril | 40 % |
| | | Methanol | 30 % |
| | | *Aqua bidest.* | 30 % |
| Flussrate: | 1 ml / min | | |

### Gradientenprofil:

| Zeit [min] | Eluent A [%] | Eluent B [%] |
|---|---|---|
| 0 | 100 | - |
| 2 | 100 | - |
| 3,5 | 90 | 10 |
| 12 | 90 | 10 |
| 16 | 25 | 75 |
| 25 | 25 | 75 |
| 28 | 100 | - |
| 35 | 100 | - |

| | |
|---|---|
| Detektion: | Fluoreszenz: *λ*ₑₓ: 338 nm; *λ*ₑₘ: 425 nm |
| Injektionsvolumen: | 10 µl |
| Derivat.-Reagenz: | 50 mg *o*-PA gelöst in 1 ml Methanol |
| | + 9 ml 0,2 M Kaliumborat-Puffer pH 9,4 |
| | + 53 µl 2-Mercaptoethanol |
| Derivatisierung: | Der Autosampler wurde so programmiert, dass 10 µl der Probe mit 14 µl des Derivatisierungs-Reagenzes gemischt wurden und 3 min lang bei Raumtemperatur vor der Injektion reagieren konnten. |

### Retentionszeiten:

| | | |
|---|---|---|
| L-Citrullin | 12,2 | ± 0,3 min |
| NMMA | 15,8 | ± 0,4 min |
| Methylamin | 23,4 | ± 0,1 min |

## Patentansprüche

1. Inhibitor der Dimethylarginin Dimethylaminohydrolase (DDAH) der allgemeinen Strukturformel 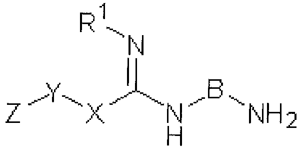 wobei
B eine verzweigte oder unverzweigte, substituierte oder nicht substituierte, gesättigte oder ungesättigte Kohlenwasserstoffkette mit einer Kettenlänge von 1 bis 6 ist,
R¹ ausgewählt ist aus der Gruppe von Strukturen (i-vii): 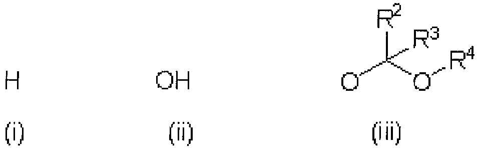 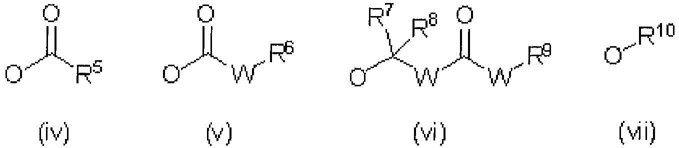
wobei
R², R³, R⁴ ausgewählt sind aus der Gruppe bestehend aus Wasserstoff, einem Alkyl- oder einem Arylrest,
R⁵, R⁶ ausgewählt sind aus der Gruppe bestehend aus Wasserstoff, einer Kohlenwasserstoffkette mit einer Kettenlänge von 1 bis 8 und einem Arylrest,
R⁷, R⁸, R⁹, R¹⁰ ausgewählt sind aus der Gruppe bestehend aus Wasserstoff, einem Alkyl- oder einem Arylrest,
W Sauerstoff (O) oder Stickstoff (N) ist, und
X eine Methylengruppe (CH₂) oder eine sekundäre Aminogruppe (NH) ist,
Y eine verzweigte oder unverzweigte, substituierte oder nicht substituierte, gesättigte oder ungesättigte Kohlenwasserstoffkette mit einer Kettenlänge von 1 bis 6 und/oder ein substituiertes oder nicht substituiertes aromatisches System mit einer Ringgröße von 3 bis 6 ist,
Z Wasserstoff (H) oder eine Methoxylgruppe ist
und der die Aminogruppe tragende Rest (B) keine Carboxylgruppe aufweist, und wobei ein Substituent von B ausgewählt ist aus der Gruppe bestehend aus einem Halogen, insbesondere Fluor, Chlor, Brom oder Jod, Schwefel, einer Alkoxy-, Amino-, Hydroxyl-, Carbonyl-, Mercapto-, Benzyloxy-, Phenyl-, Benzyl-, Cyano-, Nitro- und Thioalkoxy-Gruppe,
zur Verwendung in der Behandlung der Ischämie-Reperfusion-Verletzung des Herzens oder Gehirns, Hypotonie, (chronischen) Entzündungen, neurodegenerativen Erkrankungen, Tumorerkrankungen, Peritonitis, Asthma, Migräne, Schmerz, Sepsis oder septischem Schock oder zur Prophylaxe von Schmerzen.

2. Inhibitor zur Verwendung nach Anspruch 1, **dadurch gekennzeichnet, dass** B und/oder Y mehrfach ungesättigt sind.

3. Inhibitor zur Verwendung nach einem der vorhergehenden Ansprüche, **dadurch gekennzeichnet, dass** Y Substituenten aufweist, ausgewählt aus der Gruppe bestehend aus einem Halogen, insbesondere Fluor, Chlor, Brom oder Jod, Sauerstoff, Schwefel, einer Alkoxy-, Acyloxy-, Amino-, Hydroxyl-, Carbonyl-, Mercapto-, Carboxy-, Benzyloxy-, Phenyl-, Benzyl-, Cyano-, Nitro-, Thioalkoxy-, Carboxyaldehyd-, Carbalkoxy- und Carboxamid-Gruppe.

4. Inhibitor zur Verwendung nach einem der Ansprüche 1 bis 3 zur Verwendung als Arzneimittel in der Behandlung einer Krankheit, deren Pathologie mit einem Überangebot an endogenen Stickoxiden (NOₓ) einhergeht.

## Claims

1. Inhibitor of Dimethylarginin Dimethylaminohydrolase (DDAH) having the general structural formula 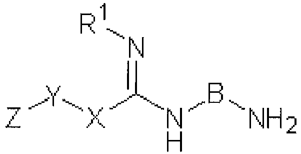 wherein
B is a linear or branched, substituted or unsubstituted, saturated or unsaturated hydrocarbon chain with a length of 1 to 6,
R¹ is selected from the group of structures (i-vii): 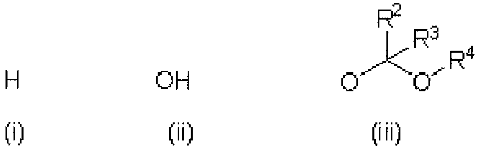 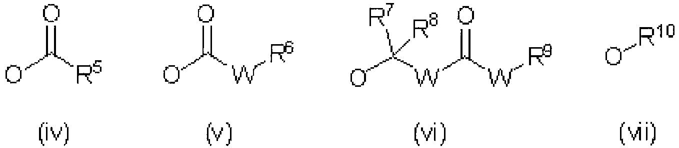
wherein
R², R³, R⁴ are selected from the group consisting of hydrogen, an alkyl or an aryl group,
R⁵, R⁶ are selected from the group consisting of hydrogen, a hydrocarbon chain with a length of 1 to 8 and an aryl group,
R⁷, R⁸, R⁹, R¹⁰ are selected from the group consisting of hydrogen, an alkyl or an aryl group,
W is oxygen (O) or nitrogen (N), and
X is a methylene group (CH₂) or a secondary amino group (NH)
Y is a linear or branched, substituted or unsubstituted, saturated or unsaturated hydrocarbon chain with a length of 1 to 6 and/or a substituted or unsubstituted aromatic system with a ring size of 3 to 6,
Z is hydrogen (H) or a methoxyl group
and in which the amino group carrying rest (B) has no carboxy group, and wherein a substituent of B is selected from the group consisting of a halogen, especially fluorine, chlorine, bromine or iodine, sulphur, an alkoxy, amino, hydroxyl, carbonyl, thiol, benzoic acid, phenyl, benzyl, cyano, nitro and thio alkoxy group,
for use in the treatment of the ischemia-reperfusion injury in heart or brain, hypotension, (chronic) inflammations, neurodegenerative diseases, tumour diseases, peritonitis, asthma, migraine, pain, sepsis or septic shock or in the prophylaxis of pain.

2. Inhibitor for use according to claim 1 **characterized by** that B and/or Y are repeatedly unsaturated.

3. Inhibitor for use according to any one of the precedent claims **characterized by** that Y has substituents selected from the group consisting of a halogen, especially fluorine, chlorine, bromine or iodine, oxygen, sulphur, an alkoxy, acyloxy, amino, hydroxyl, carbonyl, thiol, carboxyl, benzoic acid, phenyl, benzyl, cyano, nitro, thio alkoxy, carboxyl aldehyde, carbalkoxyl and carboxyl amide group.

4. Inhibitor for use according to any of claims 1 to 3 for use as a medicament for the treatment of a disease, the pathology of which is accompanied by an excess of endogenous nitrogen oxides (NOₓ).

## Revendications

1. Inhibiteur de la diméthylarginine diméthylaminohydrolase (DDAH) de formule structurale générale 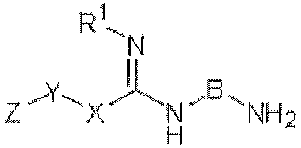 où
B est une chaîne hydrocarbonée saturée ou insaturée, substituée ou non substituée, ramifiée ou non ramifiée ayant une longueur de chaîne de 1 à 6,
R¹ est choisi dans le groupe de structures (i-vii): 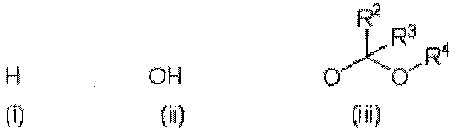 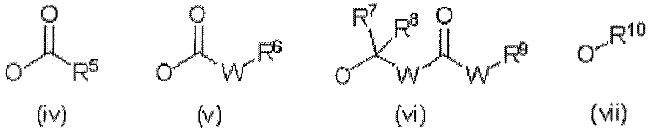 où
R², R³, R⁴ sont choisis dans le groupe consistant en l'hydrogène, un groupement alkyle ou un groupement aryle,
R⁵, R⁶ sont choisis dans le groupe consistant en l'hydrogène, une chaîne hydrocarbonée ayant une longueur de chaîne de 1 à 8 et un groupement aryle,
R⁷, R⁸, R⁹, R¹⁰ sont choisis dans le groupe consistant en l'hydrogène, un groupement alkyle ou un groupement aryle,
W est l'oxygène (O) ou l'azote (N), et
X est un groupe méthylène (CH₂) ou un groupe amino secondaire (NH),
Y est une chaîne hydrocarbonée saturée ou insaturée, substituée ou non substituée, ramifiée ou non ramifiée ayant une longueur de chaîne de 1 à 6 et/ou un système aromatique substitué ou non substitué ayant une taille de cycle de 3 à 6,
Z est l'hydrogène (H) ou un groupe méthoxyle
et le groupement (B) portant le groupe amino ne comporte aucun groupe carboxyle, et où un substituant de B est choisi dans le groupe consistant en un halogène, en particulier le fluor, le chlore, le brome ou l'iode, le soufre, un groupe alcoxy, amino, hydroxyle, carbonyle, mercapto, benzyloxy, phényle, benzyle, cyano, nitro et thioalcoxy,
destiné à être utilisé dans le traitement de la lésion d'ischémie-reperfusion du coeur ou du cerveau, de l'hypotonie, des inflammations (chroniques), des maladies neurodégénératives, des maladies tumorales, de la péritonite, de l'asthme, de la migraine, de la douleur, de la septicémie ou du choc septique ou pour la prophylaxie des douleurs.

2. Inhibiteur destiné à être utilisé selon revendication 1, **caractérisé en ce que** B et/ou Y sont plusieurs fois insaturés.

3. Inhibiteur destiné à être utilisé selon l'une des revendications précédentes, **caractérisé en ce que** Y comporte des substituants choisis dans le groupe consistant en un halogène, en particulier le fluor, le chlore, le brome ou l'iode, l'oxygène, le soufre, un groupe alcoxy, acyloxy, amino, hydroxyle, carbonyle, mercapto, carboxy, benzyloxy, phényle, benzyle, cyano, nitro, thioalcoxy, carboxyaldéhyde, carbalcoxy et carboxamide.

4. Inhibiteur destiné à être utilisé selon l'une des revendications 1 à 3 destiné à être utilisé comme médicament dans le traitement d'une maladie dont la pathologie se développe avec un excès d'oxydes d'azote (NOₓ) endogènes.
